# EUROPÄISCHE PATENTSCHRIFT

(11) **EP 1 372 518 B1**
(45) Veröffentlichungstag und Bekanntmachung des Hinweises auf die Patenterteilung: **20.07.2005**
(21) Anmeldenummer: 02724265.0
(22) Anmeldetag: 28.03.2002
(51) Int. Cl.: A61C 1/00, A61C 1/08, A61F 9/008, A61B 18/20

(54) **LASERBEHANDLUNGSGERÄT MIT BELEUCHTUNGSSYSTEM**
LASER TREATMENT DEVICE WITH A LIGHTING SYSTEM
APPAREIL DE TRAITEMENT LASER DOTE D'UN SYSTEME D'ECLAIRAGE

(30) Priorität: 06.04.2001 DE 10117347
(43) Veröffentlichungstag der Anmeldung: 02.01.2004
(73) Patentinhaber: W & H Dentalwerk Bürmoos GmbH, 5111 Bürmoos (AT)
(72) Erfinder: STRASSL, Martin, W & H Dentalwerk Bürmoos GmbH, 5111 Bürmoos (AT); BRUGGER, Wilhelm, W & H Dentalwerk Bürmoos GmbH, 5111 Bürmoos (AT); KASENBACHER, Anton, 83278 Traunstein (DE); NIEMZ, Mark, 76547 Sinzheim (DE); BAUER, Thorsten, 30167 Hannover (DE)
(74) Vertreter: Appelt, Christian W.
(86) Internationale Anmeldenummer: PCT/EP2002/003545
(87) Internationale Veröffentlichungsnummer: WO 2002/080803

(56) Entgegenhaltungen:
- EP-A- 0 844 008
- DE-A- 3 319 203
- DE-U- 29 705 934
- US-A- 5 364 390
- NIEMZ M H: "INVESTIGATION AND SPECTRAL ANALYSIS OF THE PLASMA-INDUCED ABLATION MECHANISM OF DENTAL HYDROXYAPATITE" APPLIED PHYSICS B: LASERS AND OPTICS, SPRINGER INTERNATIONAL, BERLIN, DE, Bd. B58, Nr. 4, 1. April 1994 (1994-04-01), Seiten 273-281, XP000457142 ISSN: 0946-2171

## Beschreibung

Die vorliegende Erfindung betrifft ein Laserbehandlungsgeräts mit Beleuchtungssystem, insbesondere für den Einsatz im medizinischen Bereich, wobei das Laserbehandlungsgerät eine Laserlichtquelle zur Erzeugung eines Bearbeitungslaserstrahls und eine Beleuchtungslichtquelle zur Erzeugung eines sichtbaren Lichts zur Beleuchtung eines zu bearbeitenden Bereiches und ein oder mehrere erste optische Elemente umfaßt, über das oder die der Bearbeitungslaserstrahl weitergeleitet wird.

Insbesondere aus dem zahnmedizinischen Bereich sind unterschiedliche Behandlungsgeräte bekannt, wobei sowohl Behandlungsgeräte mit mechanischen Behandlungselementen, wie z.B. einem Bohrer, aber auch Behandlungsgeräte eingesetzt werden, die anstelle der mechanischen Bearbeitungselemente einen Laserstrahl verwenden.

Je nach Anwendungsgebiet ist es vorteilhaft oder erforderlich, den zu bearbeiteten Bereich zu beleuchten, um den Bediener der Geräte die geeigneten Lichtverhältnisse zu verschaffen. Häufig ist es, insbesondere in schwer zugänglichen Bereichen, wie es insbesondere im zahnmedizinischen Bereich der Fall ist, schwierig, mit externen Beleuchtungsquellen den zu bearbeiteten Bereich auszuleuchten, da insbesondere das Behandlungs- bzw. Bearbeitungsgerät bzw. der Bediener selbst den zu bearbeiteten Bereich abschattet.

Es sind daher aus dem Stand der Technik Geräte bekannt, an denen integral mit dem Behandlungsgerät ausgebildete Beleuchtungsvorrichtungen vorgesehen sind.

Aus der DE 33 32 628 C2 ist beispielsweise ein zahnärztliches Handstück bekannt, das einen Behandlungskopf aufweist, an dem ein mechanisches Behandlungswerkzeug angebracht ist. Das Handstück weist ferner einen Lichtleiter auf, der durch das Handstück geführt ist und seitlich in der Nähe des Behandlungswerkzeug aus dem Handstück herausgeführt ist, so daß das Bearbeitungswerkzeug sowie der zu bearbeiteten Bereich mittels einer Beleuchtungslichtquelle beleuchtet werden kann.

Aus der DE 33 28 604 A1 ist ferner ein Zahnsteinentfernungs-Handstück bekannt, das, ähnlich wie bei dem in der DE 33 32 628 C2 offenbarten Gerät, in einem vorderen Bereich ein Behandlungswerkzeug, hier ein Zahnsteinentfemungs-Werkzeug, aufweist, das in Schwingungen versetzt wird. Auch bei der in dieser Druckschrift offenbarten Vorrichtung wird durch das Handstück ein Lichtleiter geführt, der in der Nähe des Bearbeitungswerkzeuges mündet und den zu bearbeiteten Bereich mittels einer Beleuchtungslichtquelle beleuchten soll.

Die bekannten Beleuchtungssysteme nach dem Stand der Technik haben den Nachteil, daß die Zuführung des Lichtleiters relativ aufwendig und damit kostenintensiv ist, wobei die Lichtleiter zusätzlich relativ empfindlich sind, was im Laufe der Lebensdauer eines entsprechenden Gerätes zu Verminderungen der zur Verfügung stehenden Beleuchtungslichtstärke führen kann. Ferner muß bei einer Zerstörung des Lichtleiters häufig der gesamte Lichtleiter ausgewechselt werden, was sehr kostenintensiv oder unter Umständen wirtschaftlich gar nicht mehr möglich ist.

Ferner ist je nach Behandlungswerkzeug die Ausleuchtung des zu bearbeitenden Bereichs nicht optimal, was insbesondere bei Bohrern der Fall ist, bei denen das Licht durch einen seitlich angebrachten Lichtleiter zugeführt wird.

Aus der DE 33 19 203 A1 ist ferner ein Verfahren und eine Vorrichtung zur Dosismessung bei der Photokoagulation bekannt, bei der ein von einem Laser kommendes monochromatisches Laserlicht einerseits und Licht einer Lampe zur Objektbeleuchtung andererseits über einen chromatischen Strahlteiler auf eine Probe gelenkt wird, wobei der chromatische Strahlteiler für das Licht der Lampe zur Objektbeleuchtung durchlässig ist, also transmittierend wirkt, während der Strahlteiler für das Laserlicht reflektierend wirkt. Ein von der Probe ausgehendes Fluoreszenzlicht wird durch einen für dieses Fluoreszenzlicht durchsichtigen Strahlteiler, der für das Licht der Lampe zur Objektbeleuchtung jedoch reflektierend ist, zumindest teilweise ausgekoppelt, so daß zumindest ein Teil des Fluoreszenzlichts mit einem Photomultiplier ausgewertet werden kann.

Aus der WO 01/19454 A2 ist ferner eine Laservorrichtung bekannt, bei der neben einer Laserdiode zur Erzeugung eines Laserlicht auch eine Diode zur Erzeugung eines sogenannten "Führungslichts" vorhanden ist. Das Licht beider Lichtquellen wird auch in diesem Fall über einen monochromatischen Strahlteiler eingekoppelt, wobei der Strahlteiler für das sogenannte Führungslicht vollständig reflektierend ist.

US-A-5 364 390 beschreibt ein Laserbehandlungsgerät gemäß dem Oberbegriff des Anspruchs 1.

Diese Systeme nach dem Stand der Technik haben insbesondere den Nachteil, daß in dem Fall, daß das bei einer Diagnose oder Analyse einer beim Bearbeitungsvorgang erzeugten Strahlung bzw. eines Spektroskopielichts einen Wellenlängenbereich aufweist, der dem Wellenlängenbereich des Beleuchtungslicht entspricht oder mit diesem überlappt, eine Diagnose oder Analyse nicht oder nur sehr schwer möglich ist, insbesondere bei einer schwachen Strahlung bzw. einem schwachen Spektroskopielicht, da das Spektroskopielicht in dem zumindest überlappenden Wellenlängenbereich nicht oder nur unzureichend ausgekoppelt werden kann.

Es ist daher eine Aufgabe der vorliegenden Erfindung, ein Laserbehandlungsgeräts mit einem Beleuchtungssystem zur Verfügung zu stellen, das die oben genannten Nachteile des Standes der Technik vermeidet und insbesondere auf einfache und kostengünstige Weise ein effektives Beleuchtungssystem und eine effektive Möglichkeit zur Diagnose oder Analyse des Bearbeitungsvorgangs zur Verfügung stellt.

Diese Aufgabe wird durch ein Laserbehandlungsgerät gemäß Anspruch 1 gelöst, die Ansprüche 2 bis 17 betreffen besonders vorteilhafte Ausführungsformen des erfindungsgemäßen Laserbehandlungsgeräts.

Erfindungsgemäß umfaßt das Laserbehandlungsgerät daher ein oder mehrere optische Mehrfunktionselemente, die alle so ausgelegt sind, daß sie den Bearbeitungslaserstrahl einerseits und das sichtbare Licht der Beleuchtung andererseits im wesentlichen vergleichbar beeinflussen, wobei hierunter zu verstehen ist, daß die Mehrfunktionselemente entweder für die Wellenlängen sowohl des Bearbeitungslaserstrahls als auch des sichtbaren Beleuchtungslicht reflektierend sind oder aber für beide Wellenlängen bzw. Wellenlängenbereiche transmittierend ausgebildet sind, so daß es sich bei den optischen Mehrfunktionselementen gemäß dieser Erfindung nicht um chromatische Strahlteiler handelt, wie sie im Stand der Technik bisher zumindest zum Einkoppeln bzw. Zusammenführen des Bearbeitungslaserlichts und des Beleuchtungslichts als erforderlich angesehen worden sind.

Durch die erfindungsgemäße Ausgestaltung des Laserbehandlungsgeräts wird daher zum einen ermöglicht, daß die ersten optischen Elemente, die sowieso erforderlich sind, um den von der Laserlichtquelle erzeugten Bearbeitungslaserstrahls auf den zu bearbeitenden Bereich zu lenken, zumindest teilweise ebenfalls dazu verwendet werden, sichtbares Licht der Beleuchtungslichtquelle auf den zu behandelnden Bereich zu lenken, zum anderen wird ermöglicht, daß eine beim Bearbeitungsvorgang erzeugte Strahlung auf effektive Weise der optischen Auswertevorrichtung, insbesondere einem Spektrometer, zugeführt wird und Verluste vermieden werden.

Darüber hinaus sind die ersten optischen Elemente zur Leitung bzw. Lenkung des Bearbeitungslaserstrahls in der Regel sehr hochwertige optische Elemente, so daß auch ein sehr effektives Weiterleiten des Beleuchtungslichtes ermöglicht wird. Solche hochwertigen Elemente werden in der Regel aus Kostengründen nicht für die Beleuchtung eingesetzt, so daß mit der vorliegenden Erfindung gleichsam ein doppelter Effekt erzeugt wird, zum einen eine Kostenreduktion, da optische Elemente eingespart werden, zum anderen eine qualitative Verbesserung, da auch für das sichtbare Beleuchtungslicht sehr hochwertige optische Elemente eingesetzt werden können.

Es soll an dieser Stelle angemerkt werden, daß die Wellenlänge des Bearbeitungslaserstrahls sich in der Regel nicht im sichtbaren Bereich befindet. Aufgrund der unterschiedlichen geometrischen Ausgestaltungen bzw. Fokussierung des Bearbeitungslaserstrahls im Vergleich zu einem gewünschten Beleuchtungslicht eignet sich aber der Laserstrahl unabhängig von seiner Wellenlänge grundsätzlich nicht zur Beleuchtung des zu bearbeitenden Bereiches, da der Bearbeitungslaserstrahl in der Regel auf einen Bearbeitungspunkt fokussiert werden muß, während das Beleuchtungslicht einen größeren Bereich um die zu bearbeitende Stelle möglichst gleichmäßig ausleuchten soll.

Gleiches gilt für eventuell vorhandene sogenannten Pilotlaserstrahlen, die Licht im sichtbaren Bereich emittieren und als "Zielvorrichtung" eingesetzt werden, um dem Bediener anzuzeigen, an welcher Stelle der ggf. für den Benutzer unsichtbare Bearbeitungslaserstrahl auf das zu bearbeitenden Material wirkt oder wirken wird.

Unabhängig von der Fokussierung würde sich ein solcher Pilotlaserstrahl auch im Falle einer optischen Aufweitung nicht als Beleuchtungslicht eignen, da es in einem solchen Falle durch die hohe Kohärenzlänge zu destruktiven Interferenzerscheinungen an der Oberfläche des zu bearbeitenden Material kommt, wodurch sogenannte "Speckles" entstehen, die als schwarze bzw. dunkle Flecken im beleuchteten Feld auftreten und damit ein ungestörtes Erkennen von Einzelheiten verhindern, was gerade durch die Beleuchtung vermieden werden soll. Das Auftreten von "Speckles" kann dazu führen, daß das Erkennen von Einzelheiten häufig deutlich schlechter ist als ohne den ggf. aufgeweiteten Pilotlaserstrahl. Gleiches gilt analog für eine ggf. vorhandene Laserlichtquelle, die zu Diagnosezwecken, bsw. zur Anregung einer Fluoreszenz eingesetzt wird.

Unter "optischen Elementen" im Sinne der Erfindung sind alle bekannten optischen Elemente im weitesten Sinne zu verstehen, sowohl Elemente, die den Verlauf des Lichts bzw. eines Laserstrahls beeinflussen, wie z.B. lenkende, fokussierende, aufweitende, reflektierende oder das auf sie einfallende Licht sonstwie beeinflussende Elemente. Ferner umfaßt der Begriff "optische Elemente" im Sinne der Erfindung auch Elemente, die den Verlauf des auf sie auftreffenden Lichts oder eines Bearbeitungslaserstrahl im wesentlichen nicht beeinflussen, wie z.B. Austritts- oder Abschlusscheiben, die für die Wellenlänge des entsprechenden Lichts im wesentlichen vollständig transmittierend sind, wobei auch keine Aus- oder Ablenkung beim Durchlauf eines solchen optischen Elementes auftritt (oder eine solche Ablenkung vernachlässigbar ist). Optische Elemente können insbesondere Spiegel, Hohlleiter, Lichtleiter, wie z.B. Glasfasern, Abdeck- oder Austrittsfenster sein.

Ferner soll darauf hingewiesen werden, daß ein optisches Element auch unterschiedliche Bereiche mit unterschiedlichen Wirkungen in diesen Bereichen umfassen kann. Ein optisches Element im Sinne der Erfindung kann z.B. ein Spiegel sein, der über einen weiten Bereich im wesentlichen eben ausgebildet ist, der aber in einem Randbereich eine Krümmung aufweist. Grundsätzlich ist es auch möglich, daß ein optisches Element zwischen den unterschiedlichen Bereichen klare Grenzen aufweist, wie z.B. Kanten oder Beschichtungen, beispielsweise auch nicht reflektierende Beschichtungen. Ferner ist es möglich, daß ein optisches Element im Sinne der Erfindung auch zweigeteilt sein kann, um die gewünschten Effekte zu erzeugen.

Als "Laserlichtquelle" im Sinne der Erfindung ist sowohl ein einfacher Laser wie z.B. ein Diodenlaser, als auch eine komplexe Laservorrichtung zu verstehen, die ggf. auch aus mehreren einzelnen Lasern bestehen kann, beispielsweise neben dem das Bearbeitunglaserlicht erzeugenden Laser einen zugehörigen Pumplaser umfaßt. Zu der Laserlichtquelle im Sinne der Erfindung können auch sämtliche zugehörigen Geräte wie z.B. Frequenzkonvertor etc. gehören.

Unter "Beleuchtungslichtquelle" im Sinne der Erfindung ist eine Lichtquelle zu verstehen, die nicht ausschließlich im wesentlichen monochromatisches Licht, wie z.B. ein Laserlicht, aussendet. Eine solche Beleuchtungslichtquelle kann aus einer oder mehreren im Prinzip beliebigen Lichtquellen bestehen, es ist jedoch auch möglich, daß als Beleuchtungslichtquelle im Sinne der Erfindung lediglich eine Vorrichtung zu verstehen ist, die Licht aus einer anderen, ggf. nicht zum Laserbehandlungsgerät gehörenden Quelle, in das Laserbehandlungsgerät einleitet bzw. einkoppelt. Dies umfaßt z.B. auch eine Spiegelvorrichtung, die Tageslicht oder Fremdlicht zur Beleuchtung nutzt, welches ebenfalls den gewünschten Anforderungen entspricht. Es ist bevorzugt, daß die Beleuchtungslichtquelle bevorzugt weißes Licht emittiert, bevorzugt zumindest aus wenigstens zwei, insbesondere wenigstens drei zentralen Wellenlängen zusammengesetzt ist, deren Kohärenzlänge passend gewählt ist, um insbesondere die oben erwähnten "Speckles" zu vermeiden, die eine effektive Beleuchtung nicht ermöglichen. Insbesondere ist ein Beleuchtungslicht gewünscht, daß eine sehr breitbandige Frequenz- bzw. Wellenlängenverteilung aufweist. Bevorzugt werden insbesondere Beleuchtungslichtquellen eingesetzt, die den Anforderungen der DIN-Normen für Arbeitsplatzbeleuchtungen entsprechen, insbesondere den DIN-Normen 5035 (insbesondere Teil 1) und 6169 (insbesondere Teil 1 und2).

Bei einer bevorzugten Ausführungsform ist das erfindungsgemäße Laserbehandlungsgerät so ausgebildet, daß der in ihm geführte Bearbeitungslaserstrahl im wesentlichen frei geführt ist. Unter freier Führung des Laserstrahls ist eine weitgehende Führung in einem im wesentlichen freien Innenraum eines Bauteiles des Laserbehandlungsgerätes zu verstehen, der mit Luft bzw. einem Gas gefüllt sein kann und ggf. auch einen Unterdruck bzw. ein Vakuum aufweisen kann. Die Führung des Laserstrahls in diesem freien Innenraum des Bauteils wird mittels der oben genannten ersten optischen Elemente, insbesondere mittels Spiegeln und Linsen sowie Fenstern gelenkt und ausgerichtet bzw. fokussiert etc.

Bei einer solchen Ausführungsform bestehen extrem viele Freiheitsgrade zur Verfügung sowohl des Bearbeitungslaserstrahls als auch zur zusätzlichen Führung des sichtbaren Lichts zur Beleuchtung ebenfalls in dem Innenraum des Bauteils unter Nutzung insbesondere möglichst vieler erster optischer Elemente geführt werden kann.

Bevorzugt umfaßt das Laserbehandlungsgerät auch ein erstes optisches Element, das im wesentlichen als für das Laserbehandlungslicht transmittierend ausgebildetes Austrittsfenster ausgebildet ist und das den Verlauf des Bearbeitungslaserstrahls im wesentlichen nicht beeinflußt. Ein solches Austrittsfenster wird in der Regel zum Abschluß des bearbeitungsseitigen Endes des Bauteils, insbesondere des Handstücks, vorgesehen, um den Innenraum nach außen abzuschirmen. Wird das Beleuchtungslicht ebenfalls durch dieses Austrittsfenster geführt, so wird insbesondere eine sehr effektive Beleuchtung realisiert, da das sichtbare Licht zur Beleuchtung im wesentlichen aus der gleichen Richtung und parallel sowie bevorzugt im gleichen Raumbereich wie der Behandlungslaserstrahl abgestrahlt, so daß auch bei sehr schwer zugänglichen Bereichen, insbesondere im zahnmedizinischen Bereich, als Beispiel seien hier insbesondere Wurzelbehandlungen genannt, eine Abschattung vermieden wird.

Neben der beschriebenen Ausfühnmgsform, bei der das Austrittsfenster des Laserbehandlungsgerätes als optisches Merhfunktionselement ausgebildet ist, ist selbstverständlich auch eine Ausführungsform denkbar, bei der zusätzlich zu dem Austrittsfenster auch ein Eintrittsfenster eines solchen Laserbehandlungsgerätes als optisches Mehrfunktionselement zur Weiterleitung oder Lenkung sowohl des Behandlunglaserstrahls als auch des sichtbaren Lichts zur Beleuchtung ausgebildet sein kann.

Besonders bevorzugt ist es jedoch, daß insbesondere solche erste optische Elemente als optische Mehrfunktionselemente ausgebildet sind, die nicht lediglich einen bestimmten Raumbereich abschirmen und den Bearbeitungslaserstrahl und/oder das sichtbare Licht zur Beleuchtung im wesentlichen nicht beeinflussen, sondern die als den Bearbeitungslaserstrahl und/oder das sichtbare Licht zur Beleuchtung beeinflussende Elemente ausgebildet sind. Dazu zählen insbesondere Linsen, die durch sie hindurchfallendes Licht fokussieren, aufweiten oder sonstwie beeinflussen, oder Spiegelelemente, die den Laserstrahl in die gewünschte Richtung insbesondere im Innenbereich des Bauteiles bei einem frei geführten Laserstrahl lenken, wobei solche Spiegel ebenfalls fokussierende oder aufweitende Funktionen aufweisen können. Dadurch wird insbesondere ermöglicht, daß das sichtbare Licht zur Beleuchtung einen Weg durch das Bauteil nehmen kann, der zumindest in weiten Bereichen ähnlich verläuft wie der vorbestimmte Weg des Bearbeitungslaserstrahls. Zusätzliche Elemente zur Lenkung oder Beeinflussung des sichtbaren Lichtes sind nicht erforderlich.

Je mehr der ersten optischen Elemente für den Bearbeitungslaserstrahl als optische Mehrfunktionselemente ausgebildet sind, desto weniger zusätzliche optische Elemente sind erforderlich, die lediglich für das sichtbare Licht zur Beleuchtung vorgesehen sind.

Bei einer weiteren bevorzugten Ausführungsform des erfindungsgemäßen Laserbehandlungsgerätes umfaßt dieses ein optisches Element zum Einkoppeln des Beleuchtungslichts in das Bauteil. Ein solches optisches Element ist dann erforderlich, wenn die Beleuchtungslichtquelle nicht in dem besagten Bauteil vorgesehen ist, es sich bei dem Bauteil daher um ein Mittelstück oder um ein bearbeitungsseitiges Endstück, insbesondere das Handstück handelt, wobei die Beleuchtungslichtquelle in Elementen oder Bauteilen vorgesehen ist, die weiter von dem bearbeitungsseitigen Ende der Vorrichtung entfernt sind. Dies ist insbesondere deshalb vorteilhaft, weil gerade die bearbeitungsseitigen Endstücke, insbesondere das Handstück, zumindest bei medizinischen Bereichen häufig sterilisiert werden müssen, wobei es günstig ist, empfindliche oder schwierig zu sterilisierende Elemente in einen Bereich zu legen, der nicht so regelmäßig sterilisiert werden muß.

Bevorzugt ist dieses Element zum Einkoppeln des Beleuchtungslichtes so ausgebildet, daß auch der Bearbeitungs- bzw. Behandlungsstrahl über das gleiche optische Element eingekoppelt werden kann, so daß es sich auch bei diesem Element um ein optisches Mehrfunktionselement im Sinne dieser Erfindung handelt.

Für das Anordnen der Laserlichtquelle in dem Laserbehandlungsgerät gilt in noch viel stärkerem Maße das oben gesagte, da es sich bei der Laserlichtquelle in der Regel um komplexe und auch anfällige Vorrichtungen handelt, so daß diese bevorzugt in "hinteren" Bauteilen, also entfernt von dem bearbeitungsseitigen Ende, angeordnet sind.

Bei einer anderen Ausführungsform ist es jedoch auch möglich, daß das Bauteil selbst die Beleuchtungslichtquelle und/oder die Laserlichtquelle umfaßt. Dies kann insbesondere im Falle von Vorrichtungen der Fall sein, wo beispielsweise als Laserlichtquelle ein einfacher Diodenlaser eingesetzt werden kann. Es ist jedoch auch möglich, daß es sich bei einem solchen Bauteil um ein Bauteil handelt, daß relativ weit von dem bearbeitungsseitigen Ende des Laserbehandlungsgeräts entfernt ist, so daß das Vorsehen der Beleuchtungslichtquelle und/oder der Laserlichtquelle in diesem Bauteil unproblematisch ist.

Die Vorteile der vorliegenden Erfindung werden insbesondere dann sehr gravierend, wenn es sich bei dem Bauteil um ein Handstück, also um das bearbeitungsseitige Endstück eines Laserbehandlungsgerätes handelt, da insbesondere in diesem Bereich auf jeden Fall sowohl der Bearbeitungslaserstrahl als auch das Beleuchtungslicht zur Verfügung stehen müssen. Insbesondere ist ein solches Handstück so ausgebildet, daß vor allem die bearbeitungsseitigen optischen Elemente als optische Mehrfunktionselemente ausgebildet sind, so daß insbesondere in diesen Endbereich kurz vor dem zu behandelnden Bereich diese optischen Mehrfunktionselemente genutzt werden, was insbesondere auch zu einer sehr effektiven gleichgerichteten Beleuchtung führt. Unter "gleichgerichteter Beleuchtung" ist hier zu verstehen, daß sowohl der Bearbeitungslaserstrahl als auch das sichtbare Licht zur Beleuchtung im wesentlichen aus der gleichen Richtung und zumindest teilweise überlappend auf den zu behandelnden Bereich eingestrahlt wird, wodurch Abschattungen weitestgehend oder sogar vollständig vermieden werden.

Bevorzugt ist vorgesehen, daß zumindest das letzte der ersten optischen Elemente in Richtung des Verlaufs des Bearbeitungslaserstrahls als Mehrfunktionselement ausgebildet ist. Dieses letzte optische Element ist in der Regel das oben beschriebene Austrittsfenster.

Bei einer weiteren besonderes bevorzugten Ausführungsform ist zumindest das letzte der ersten optischen Elemente, das den Verlauf des Behandlungslaserstrahls noch beeinflußt, als optisches Mehrfunktionselement ausgebildet. Hierdurch wird insbesondere sichergestellt, daß dieses letzte, den Bearbeitungslaserstrahl lenhende oder beeinflussende optische Element, beispielsweise einen Spiegel oder eine Linse, gleichzeitig auch für die Beleuchtung genutzt wird, wodurch der oben beschriebene Effekt der gleichgerichteten Abstrahlung und der effektiven und abschattungsfreien Beleuchtung sichergestellt wird.

Als Beleuchtungslichtquelle kann auch eine "weiße Leuchtdiode" vorgesehen sein, die Licht unterschiedlicher Wellenlänge kombiniert (insbesondere Kombination einer roten, einer grünen und einer blauen LED), so daß die gewünschte gleichmäßige weißte Beleuchtung sichergestellt wir. Insbesondere werden durch eine solche "weiße Leuchtdiode" die oben beschriebenen "Speckles" vermieden, die bei einem monochromatischen Laserlicht auftreten, wie z.B. bei den oben erläuterten Pilotstrahlen. Ferner können prinzipiell standardübliche Beleuchtunglichtquellen, wie z.B. Glühlampen oder Glimmlampen eingesetzt werden.

Diese und weitere Eigenschaften und Vorteile der vorliegenden Erfindung werden anhand der nachfolgenden schematischen Zeichnungen noch erläutert. Es zeigen:
Figur 1: eine erste Ausführungsform eines erfindungsgemäßen Laserbehandlungsgeräts;
Figur 2: eine zweite Ausführungsform eines erfindungsgemäßen Laserbehandlungsgeräts;
Figur 3, eine dritte Ausführungsform eines erfindungsgemäßen Laserbehandlungsgeräts.

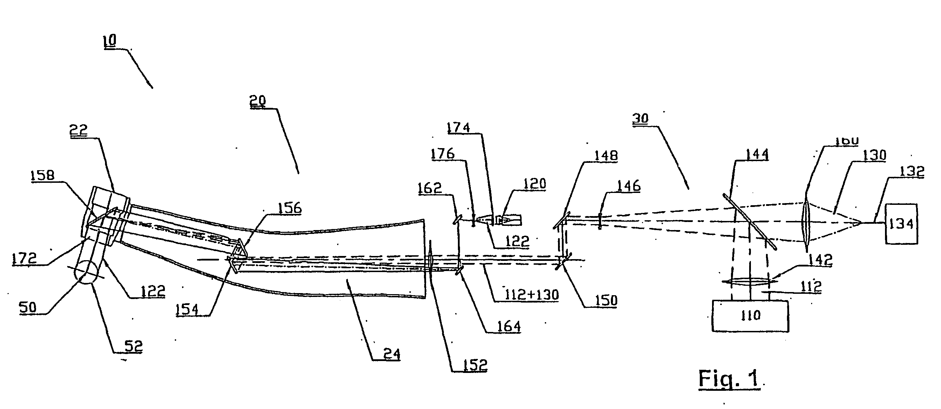

Figur 1 zeigt schematisch eine erste Ausführungsform eines erfindungsgemäßen Laserbehandlungsgeräts 10 mit einem Bauteil 20 und einem Bauteil 30 des Laserbehandlungsgeräts 10. Bei dem Bauteil 20 handelt es sich um das Handstück, das Bauteil 30 ist in seinen Abgrenzungen nicht genauer spezifiziert und kann direkt an das Handstück 20 angebracht werden oder aber auch beabstandet von diesem sein, wobei zwischen den Bauteilen 20 und 30 weitere in Figur 1 nicht gezeigte Bauteile vorhanden sein können.

Es soll an dieser Stelle angemerkt werden, daß Figur 1 die Bauteile 20 und 30 lediglich schematisch darstellt und daß in einem Betriebszustand sämtliche Bauteile 20, 30 und ggf. weitere Bauteile miteinander verbunden sind.

Die Laserlichtquelle 110 emittiert einen Bearbeitungslaserstrahl 112, der über ein System erster optischer Elemente durch das Bauteil 30 und das Handstück 20 auf einen zu bearbeitenden Bereich 50 gelenkt wird. Dabei durchläuft der Bearbeitungslaserstrahl 112 eine fokussierende Linse 142, wird von einem chromatischen Spiegelelement 144 in einem Winkel von etwa 90° reflektiert, durchläuft dann eine Linse 146 und wird dann von zwei weiteren Spiegel 148 und 150 in Richtung auf das Handstück 20 gerichtet. Die Spiegel 148 und 150 sind beweglich und bilden eine Scannvorrichtung, so daß der Bearbeitungslaserstrahl so gelenkt werden kann, daß er auf der bearbeitenden Oberfläche ein vorgegebenes Muster in einer vorgegebenen Zeit abtastet. Grundsätzlich ist es auch möglich, anstelle der zwei beweglichen Spiegel 148 und 150, die jeweils lediglich um eine Achse schwenkbar sind, wobei die Schwenkachse im wesentlichen senkrecht zueinander angeordnet sind, einen fest montierten Spiegel und nur einen um zwei Achsen beweglichen Spiegel vorzusehen.

Der vom Spiegel 150 reflektierte Bearbeitungslaserstrahl 112 wird über eine Linse 152, die bereits zum Handstück 20 gehört, in einen Innenraum 24 des Handstücks 20, in dem der Bearbeitungslaserstrahl 112 frei geführt wird, gelenkt. Grundsätzlich ist es auch möglich, daß die Linse 152 nicht zu dem Handstück 20 gehört, sondern Teil einer Kupplung bzw. eines vor dem Handstück angeordneten Elementes ist.

Da das Handstück 20 zur besseren ergonomischen Handhabung in Richtung auf sein bearbeitungsseitiges Ende gebogen ausgebildet ist, sind im Innenraum 24 des Handstücks 20 zwei weitere Spiegel 154 und 156 vorgesehen, die den Bearbeitungslaserstrahl 112 auf einen Spiegel 158, der sich im Kopfteil 22 des Bauteils 20 befindet, lenkt. Der Spiegel 158 ist das letzte lichtlenkende optische Element, das den Bearbeitungslaserstrahl 112 durch ein Lichtaustrittsfenster 172 auf den zu bearbeitenden Bereich 50 lenkt.

Das Lichtaustrittsfenster 172 ist auch ein optisches Element im Sinne der Erfindung, wobei dieses optische Element jedoch im wesentlichen keinen Einfluß auf den Verlauf des Bearbeitungslaserstrahls 112 hat.

Die in Figur 1 gezeigte Ausführungsform des Laserbehandlungsgeräts 10 umfaßt ferner eine Beleuchtungslichtquelle 120, die in dieser Ausführungsform durch eine weiße Leuchtdiode gebildet ist.

Die oben erläuterten ersten optischen Elemente 152, 154, 156, 158 und 172 sind daher als optische Mehrfunktionselemente ausgebildet, die neben dem Bearbeitungslaserstrahl 112 auch das sichtbare Licht 122 zur Beleuchtung weiterleiten.

Es soll an dieser Stelle angemerkt werden, daß sich der Bearbeitungslaserstrahl 112 und das sichtbare Licht 122 aufgrund der unterschiedlichen Wellenlängen nicht gegenseitig beeinflussen. Die optischen Mehrfunktionselemente 152, 154, 156, 158 und 172 sind dabei so ausgelegt, daß sie sowohl Licht der Wellenlänge des Bearbeitungslaserstrahls 112 als auch Licht der Wellenlänge des sichtbaren Lichts 220 vergleichbar beeinflussen, d.h. die Spiegel 154, 156, 158 sind reflektierend für beide Wellenlängen bzw. Wellenlängenbereiche, während die Linse 152 und das Lichtaustrittsfenster 172 transmittierend für beide Wellenlängen ausgebildet sind.

Als Laserlichtquelle 110 können unterschiedlichste Laser eingesetzt werden, insbesondere auch gepulste Laser, die bevorzugt ein Licht einer Wellenlänge zwischen 900 nm und 1100 nm, bevorzugt 1030 nm oder 1064 nm abstrahlen. Die Laserlichtquelle 110 ist aber in keiner Weise weder auf eine bestimmte Wellenlänge noch auf ein bestimmtes Lasersystem eingeschränkt. Das gewünschte Lasersystem und die gewünschte Wellenlänge des von der Laserlichtquelle 110 abgestrahlten Bearbeitungslaserstrahls hängt entscheidend von den gewünschten Einsatzzweck ab. Es soll an dieser Stelle nochmals angemerkt werden, daß unter der Laserlichtquelle 110, die in Figur 1 lediglich schematisch dargestellt ist, auch ein komplexes System mit mehreren Lasern, beispielsweise einschließlich Pumplasern, zu verstehen ist.

Die Laserlichtquelle 110 kann ferner Frequenzkonverter oder unterschiedliche weitere optische Elemente umfassen.

Die Beleuchtungslichtquelle 120 (hier eine standardübliche Glühlampe) ist (in Abstrahlrichtung gesehen) parallel zu dem Bearbeitungslaserstrahl 112 angeordnet, wobei das sichtbare Licht 122 zur Beleuchtung von der Beleuchtungslichtquelle 120 eine Linse 174 und eine weitere Linse 176 durchläuft und dann von zwei Spiegel 162 und 164 in die Linse 152 des Handstücks 20 eingekoppelt wird. Die Linsen 174 und 176 bringen bei dieser Ausführungsform des Beleuchtungslicht auf die passende Divergenz und regeln damit den gewünschten Durchmesser, um den Arbeitsbereich wunschgemäß auszuleuchten.

Die Linse 152 ist daher das erste optische Mehrfunktionselemant, das sowohl zur Lenkung bzw. Beeinflussung des Bearbeitungslaserstrahls 112 als auch zur Lenkung des sichtbaren Lichts 122 verwendet wird.

Die Spiegel 162, 164, 154, 156 und 158 sind dabei so ausgebildet, daß, obwohl das sichtbare Licht 122 im wesentlichen parallel zu dem Bearbeitungslaserstrahl 112 über die Linse 152 in das Handstück 20 eingekoppelt wird, der Bearbeitungslaserstrahl 112 und das sichtbare Licht 122 gleichgerichtet von dem Spiegel 158 auf den zu bearbeitenden Bereich 50 gelenkt werden.

Der Bearbeitungslaserstrahl 112 ist sehr stark fokussiert, während das sichtbare Licht 122 einen beleuchteten Bereich 52 ausleuchtet, der im wesentlichen kreisförmig ausgebildet ist, wobei der Mittelpunkt des Kreises im wesentlichen mit dem zu bearbeitenden Bereich 50 deckt. Die Größe des beleuchteten Bereichs 52 kann je nach Anwendungsbereich beliebig ausgewählt werden, im Bereich der Zahnmedizin wird dieser Bereich 52 einen Durchmesser von circa 5-10 mm, bevorzugt 6 mm aufweisen.

Das erfindungsgemäße Laserbehandlungsgerät 10 hat den Vorteil, daß neben der Bearbeitung und der Behandlung auch eine Diagnose bzw. Analyse des zu bearbeitenden Bereiches bzw. eine Analyse bei der Bearbeitung stattfinden kann.

Insbesondere im zahnmedizinischen Bereich ist es möglich, daß bei der Abtragung von Zahnmaterial entstehende Plasma spektroskopisch zu untersuchen, ohne eine zusätzliche Laserlichtquelle vorzusehen.

Bei der hier gezeigte Ausführungsform wird die durch das Plasma erzeugte auch als Spektroskopielicht bezeichnete Strahlung 130 von dem zu bearbeitenden Bereich in rückwärtiger Richtung über die optischen Elemente 172, 158, 156, 154, 152, 150, 148, 146 auf den Spiegel 144 geworfen. Der Spiegel 144 ist dabei so ausgelegt, daß er für das breitbandige Spektrum des Spektroskopielichts im wesentlichen durchlässig ist, so daß die Strahlung 130 bzw. das Spektroskopielicht durch den Spiegel 144 auf eine Linse 160 geworfen wird, die die Strahlung 130 bzw. das Spektroskopielicht fokussiert und über einen Lichtleiter 132 einer optischen Auswerteeinheit, hier einem Spektrometer 134, zuleitet.

Im Spektrometer 134 wird das Spektroskopielicht bzw. die Strahlung 130 ausgewertet, wobei hierzu selbstverständlich an dem Spektrometer Auswerteeinheiten, beispielsweise Computer etc., angeschlossen werden können.

Für eine Handstück, das eine solche Spektroskopie ermöglicht, wird auf die parallele Anmeldung DE 101 15 426 verwiesen.

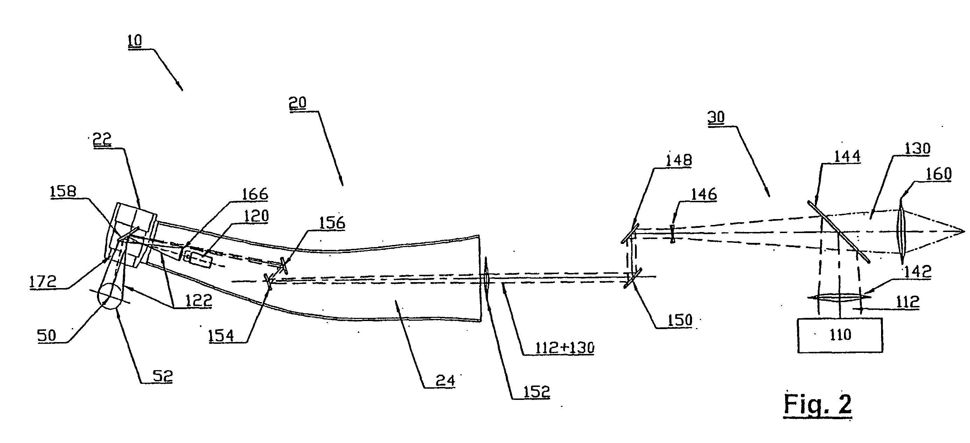

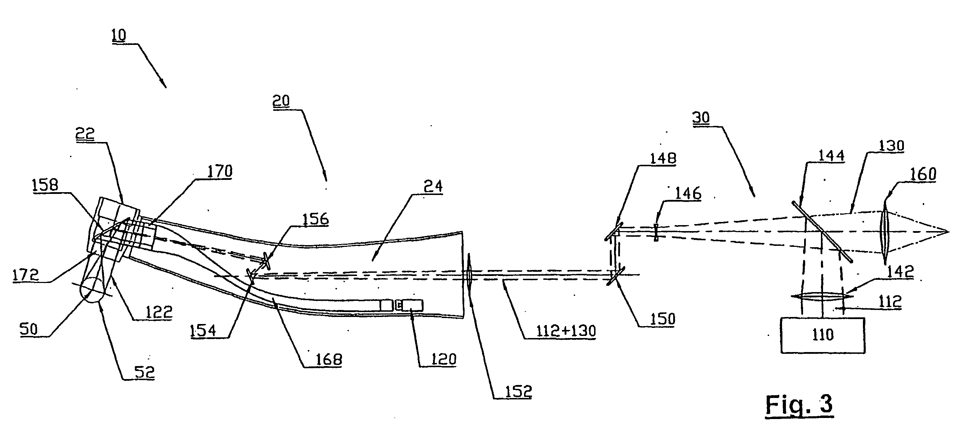

Ein weiterer Vorteil bei der in Fig. 1 gezeigten Ausführungsform ist, daß das Beleuchtungslicht nicht über die als Scanner ausgebildeten Spiegel 148, 150 geleitet wird, so daß das Beleuchtungslicht durch diese nicht beeinflußt wird. So wird ein evtl. auftretendes Flackern des Beleuchtungslichtes vermieden. Gleiches gilt für die in den Figuren 2 und 3 gezeigten Ausführungsformen.

Figur 2 zeigt eine weitere Ausführungsform eines erfindungsgemäßen Laserbehandlungsgeräts 10. Gleiche oder ähnliche Elemente sind mit identischen Bezugszeichen versehen.

Der von der Laserlichtquelle 110 emittierte Bearbeitungslaserstrahl 112 durchläuft das Laserbehandlungsgerät 10 auf analoge Weise, wie sie in Zusammenhang mit Figur 1 beschrieben worden ist.

Im Gegensatz zu den in der Figur 1 gezeigten Ausführungsformen ist bei der in Figur 2 gezeigten Ausführungsform des Laserbehandlungsgeräts 10 die Beleuchtungslichtquelle 120 in dem Handstück 20 selbst angeordnet, wobei das emittierte sichtbare Licht 122 eine Linse 166 durchläuft, bevor es auf den Spiegel 158 geworfen wird, der das sichtbare Licht 122 so reflektiert, daß es den Bereich 52 ausleuchtet. Bei dieser Ausführungsform sind lediglich der Spiegel 158, also das letzte lichlenkende bzw. lichtbeeinflussende optische Element, und das Lichtaustrittsfenster 172 als optische Mebrfunktionselemente ausgebildet, die sowohl den Bearbeitungslaserstrahl 112 als auch das sichtbare Licht 120 zur Beleuchtung weiterleiten. Bei dieser besonders vorteilhaften Ausführungsform werden daher lediglich die letzten bearbeitungsseitigen Elemente 158, 172 als optische Mehrfunktionselemente ausgebildet. Aufgrund der Anordnung der Beleuchtungslichtquelle 120 im Bereich des bearbeitungsseitigen Endes des Handstücks 20 sind auch bei dieser Ausführungsform nahezu keine zusätzlichen optischen Elemente ausschließlich für das sichtbare Licht 122 erforderlich. Lediglich die Linse 166 ist ein zweites optisches Element, das ausschließlich für das sichtbare Licht 122 vorgesehen ist.

Auch bei dieser Ausführungsform ist die in Zusammenhang mit Figur 1 erläuterte Spektroskopie möglich, in diesem Zusammenhang wird zur Vermeidung von Wiederholungen auf die Beschreibung zu Figur 1 verwiesen.

Eine noch weitere Ausführungsform des erfindungsgemäßen Laserbehandlungsgeräts 10 ist in Figur 3 dargestellt, wobei dieser Ausführungsform der in Figur 2 gezeigten Ausführungsform am ähnlichsten ist.

Auch bei der in Figur 3 gezeigten Ausführungsform ist die Beleuchtungslichtquelle 120 (hier ebenfalls eine Glühbirne) in dem Handstück 20 untergebracht, wobei das sichtbare Licht 122 zur Beleuchtung zumindest anfänglich nicht frei geführt ist, sondern über einen Lichtleiter 168 in die Nähe des Kopfteils 22 gelenkt wird.

Der Lichtleiter 168 besteht bei dieser Ausführungsform aus einem Faserpaket, das kurz vor dem Kopfteil 22 in einem im wesentlichen ringkreisförmigen Faserbündelende 170 endet, in dessen Innenraum der Bearbeitungslaserstrahl 112 verläuft. Grundsätzlich ist es natürlich auch möglich, ein kreisförmiges Faserbündel vorzusehen, wobei im wesentlichen die gesamte Kreisfläche mit Faserenden ausgefüllt ist. In diesem Fall endet dieses kreisförmige Faserbündel parallel zum Bearbeitungslaserstrahl.

Von dem Faserbündelende 170 wird das sichtbare Licht 122 zur Beleuchtung über den als optisches Mehrfunktionselement ausgebildeten Spiegel 158 auf den zu beleuchtenden Bereich 52 gelenkt, wobei das Austrittsfenster 172 durchlaufen wird.

Auch bei dieser Ausführungsform sind zumindest der Spiegel 158 sowie das Lichtaustrittsfenster 172 als optisches Mehrfunktionselement ausgebildet, so daß insbesondere eine Beleuchtung erzielt wird, die mit dem Verlauf des Bearbeitungslaserstrahls 112 gleichgerichtet ist, so daß eine abschattungsfreie Beleuchtung des zu bearbeitenden Bereichs 50 sichergestellt wird. Ferner ist der empfindliche Lichtleiter 168 und das empfindliche Faserbündelende 170 vollständig im Innenraum 24 des Handstücks 20angeordnet, so daß diese gegen äußere Einflüsse und damit gegen Beschädigung geschützt werden.

Dem Fachmann steht es frei, weitere mögliche Ausführungsformen, insbesondere Kombinationen aus den in den Figuren 1 bis 3 dargestellten Ausführungsformen, zu verwirklichen, ohne vom Gegenstand der Erfindung abzuweichen. Je nach Anwendungsgebiet wird der Fachmann das Laserbehandlungsgerät sowie die einzelnen Bauteile entsprechend den Anforderungen gestalten.

Insbesondere die in Figur 1 gezeigte Ausführungsform hat den Vorteil, daß in dem Bauteil 20, dem Handstück, keine stromführenden Teile und vor allem keine übermäßigen empfindlichen oder teuren optischen Komponenten vorhanden sind, so daß zum einen eine hohe Arbeitssicherheit gewährleistet ist, zum anderen das Handstück 20 bei den häufig erforderlichen Sterilisationen im Falle eines Einsatzes im medizinischen Bereich nicht übermäßig beansprucht wird.

Die in Figur 3 gezeigte Ausführungsform hat insbesondere den Vorteil, daß auch mehrere Lichtquellen ringförmig um die Achse des Bearbeitungslaserstrahls 112 angeordnet werden kann, wobei das Licht der mehreren Lichtquellen in einzelnen Lichtleitern oder in einzelnen Lichtfaser des Lichtleiters 168 geführt werden kann. Eine solche ringförmige Anordnung mehrerer Beleuchtungselemente ist grundsätzlich auch in allen anderen gezeigten Ausführungsformen einsetzbar.

Selbstverständlich ist an stelle der in allen Figuren gezeigten ergonomischen, gebogenen Ausführungsform des Handstückes auch ein gerades Handstück einsetzbar. Insbesondere ist es auch möglich, den Bearbeitungslaserstrahl sowie das sichtbare Licht zur Beleuchtung in Achsrichtung des bearbeitungsseitigen Elementes abzustrahlen, also nicht, wie in den Ausführungsformen gezeigt, in einem 90° Winkel, obwohl eine solche Ausgestaltung insbesondere für zahnmedizienische Behandlungen vorteilhaft ist. Selbstverständlich sind auch anderen Winkelanordnungen denkbar, diese variieren je nach Einsatz- und Anwendungsgebiet.

Abschließend soll noch darauf hingewiesen werden, daß es grundsätzlich auch möglich ist, das Laserbehandlungsgerät zusätzlich mit einem Pilotlaser auszustatten, der, wie oben erläutert, als "Zielvorrichtung" eingesetzt werden kann. Grundsätzlich ist es auch möglich, eine weitere monochromatische Lichtquelle (Diagnoselaserstrahl) im Laserbehandlungsgerät vorzusehen, wenn beispielsweise eine Analyse des zu behandelnden Materials mittels Fluoreszenz gewünscht ist. Das monochromatische Licht zur Erzeugung der Fluoreszenz kann ebenfalls, falls gewünscht, über die optischen Elemente und auch über die Mehrfunktionselemente gemäß dieser Erfindung geführt und gelenkt werden. Auch eine Kombination eines Lasers als Pilotlasers und als Diagnoselaser ist denkbar, da eine Fluoreszenz auch mit einem Laserstrahl, der im sichtbaren Wellenlängenbereich emittiert, angeregt werden kann.

Die in der vorliegenden Beschreibung, den Ansprüchen und den Zeichnungen offenbarten Merkmale können sowohl einzeln als auch in beliebiger Kombination für die Realisierung der Erfindung in ihren verschiedenen Ausgestaltungen von Bedeutung.

### Bezugszeichenliste

- 10: Laserbehandlungsgerät
- 20: Bauteil eines Laserbehandlungsgeräts (Handstück)
- 22: Kopf (Bauteil)
- 24: Innenraum (Bauteil)
- 30: Bauteil eines Laserbehandlungsgeräts
- 50: zu bearbeitender Bereich
- 52: beleuchteter Bereich
- 110: Laserlichtquelle
- 112: Bearbeitungslaserstrahl
- 120: Beleuchtungslichtquelle
- 122: sichtbares Licht (Beleuchtung)
- 130: bei der Bearbeitung erzeugte Strahlung
- 132: Lichtleiter
- 134: optische Auswertevorrichtung
- 142: Linse
- 144: Spiegel
- 146: Linse
- 148: Spiegel
- 150: Spiegel
- 152: Linse
- 154: Spiegel
- 156: Spiegel
- 158: Spiegel
- 160: Linse
- 162: Spiegel
- 164: Spiegel
- 166: Linse
- 168: Lichtleiter
- 170: Faserbündelende
- 172: Lichtaustrittsfenster
- 174: Linse
- 176: Linse

## Patentansprüche

1. Laserbehandlungsgerät mit Beleuchtungssystem, insbesondere für den Einsatz im medizinischen Bereich, das eine Laserlichtquelle (110) zur Erzeugung eines im wesentlichen frei geführten Bearbeitungslaserstrahls (112) sowie eine Beleuchtungslichtquelle (120) zur Erzeugung eines sichtbaren Lichts (122) zur Beleuchtung eines zu bearbeitenden Bereichs (50) und ein oder mehrere erste optische Elemente (142, 144, 146, 148, 150, 152, 154, 156, 158, 172) umfaßt, über die der Bearbeitungslaserstrahl (112) weitergeleitet wird, wobei mindestens eines der ersten optischen Elemente (142, 144, 146, 148, 150, 152, 154, 156, 158, 172) als optisches Mehrfunktionselement (152, 154, 156, 158, 172) und so ausgebildet ist, daß das sichtbare Licht (122) zur Beleuchtung zumindest auch über dieses mindestens eine optische Mehrfunktionselement (152, 154, 156, 158, 172) gelenkt wird,
**dadurch gekennzeichnet, daß**
das Laserbehandlungsgerät ferner eine optische Auswertevorrichtung (134) für eine Diagnose oder Analyse einer beim Bearbeitungsvorgang erzeugten Strahlung (130) umfaßt und alle optischen Mehrfunktionselemente (152, 154, 156, 158, 172) so ausgelegt sind, daß sie den Bearbeitungslaserstrahl (112) und das sichtbare Beleuchtungslicht (122) im wesentlichen gleichwirkend reflektierend und/oder transmittierend beeinflussen, und das Laserbehandlungsgerät ferner einen sich zumindest über den gesamten Querschnitt des Bearbeitungslaserstrahls (112) erstreckenden Strahlteiler (144) zum Auskoppeln der Strahlung (130) umfaßt, der so zwischen der Laserlichtquelle und dem in Einfallsrichtung des Bearbeitungslaserstrahls (112) ersten Mehrfunktionselement (152; 158) angeordnet ist, daß er von dem sichtbaren Licht (122) der Beleuchtungslichtquelle (130) nicht durchlaufen wird.

2. Laserbehandlungsgerät nach Anspruch 1, **dadurch gekennzeichnet**, das es so ausgelegt ist, daß der Bearbeitungslaserstrahl (112) in ihm im wesentlichen frei geführt ist

3. Laserbehandlungsgerät nach Anspruch 1 oder 2, **dadurch gekennzeichnet, daß** es ein erstes optisches Element umfaßt, das im wesentlichen als für das Laserbehandtunglicht transmittierendes Lichtaustrittsfenster (172) ausgebildet ist, das den Verlauf des Bearbeitungslaserstrahls (112) im wesentlichen nicht beeinflußt.

4. Laserbehandlungsgerät nach Anspruch 3, **dadurch gekennzeichnet, daß** zumindest das Lichtaustrittsfenster (172) als optisches Mehrfunktionselement ausgebildet ist.

5. Laserbehandlungsgerät nach einem der vorhergehenden Ansprüche, **dadurch gekennzeichnet, daß** es mindestens ein erstes optisches Element (152, 154, 156, 158) umfaßt, das den Verlauf des Bearbeitungslaserstrahls (112) beeinflußt.

6. Laserbehandlungsgerät nach Anspruch 5, **dadurch gekennzeichnet, daß** zumindest eins der den Bearbeitungslaserstrahl beeinflussenden ersten optischen Elemente (152, 154, 156, 158) als optisches Mehrfunktionselement (152, 154, 156, 158) ausgebildet ist.

7. Laserbehandlungsgerät nach Anspruch 5 oder 6, **dadurch gekennzeichnet, daß** zumindest eins der den Bearbeitungslaserstrahls beeinflussenden ersten optischen Elemente ein Spiegelelement (154, 156, 158) ist

8. Laserbehandlungsgerät nach Anspruch 5 bis 7, **dadurch gekennzeichnet, daß** zumindest eins der den Bearbeitungslaserstrahls beeinflussenden ersten optischen Elemente eine Fokussierlinse (152) ist.

9. Laserbehandlungsgerät nach einem der vorhergehenden Ansprüche, **dadurch gekennzeichnet, daß** alle ersten optischen Elemente als optische Mehrfunktionselemente ausgebildet sind.

10. Laserbehandlungsgerät nach einem der vorhergehenden Ansprüche, **dadurch gekennzeichnet, daß** es ein Bauteil (20) aufweist, das mindestens ein optisches Element (152) zum Einkoppeln des Beleuchtungslicht (122) in das Bauteil (20) umfaßt.

11. Laserbehandlungsgerät nach Anspruch 10, **dadurch gekennzeichnet, daß** das optische Element (152) zum Einkoppeln des Beleuchtungslicht (122) in das Bauteil so ausgebildet ist, daß auch der Behandlungslaserstrahl (112) über dieses Element (152) in das Bauteil (20) einkoppelbar ist.

12. Laserbehandlungsgerät nach einem der vorhergehenden Ansprüche, **dadurch gekennzeichnet, daß** es ein Bauteil aufweist, das selbst die Beleuchtungslichtquelle (120) und/oder die Laserlichtquelle umfaßt.

13. Laserbehandlungsgerät nach einem der Ansprüche 10 bis 12, **dadurch gekennzeichnet, daß** das Bauteil einen bearbeitungsendseitiges Handstück (20) ist.

14. Laserbehandlungsgerät nach Anspruch 13, **dadurch gekennzeichnet, daß** das Handstück (20) so ausgebildet ist, daß das sichtbare Licht (122) zur Beleuchtung im wesentlichen gleichgerichtet mit dem Bearbeitungslaserstrahl (112) aus dem Handstück (20) austritt.

15. Laserbehandlungsgerät nach einem der vorhergehenden Ansprüche, **dadurch gekennzeichnet, daß** es so ausgebildet sind, daß die Strahlachse des Bearbeitungslaserstrahls (112) einerseits und die Strahlachse des sichtbaren Lichts (122) zur Beleuchtung andererseits zumindest bei dem im Abstrahlrichtung ersten optischen Mehrfunktionselement (152; 158) zueinander versetzt sind.

16. Laserbehandlungsgerät nach einem der vorhergehenden Ansprüche, **dadurch gekennzeichnet, daß** als Beleuchtungslichtquelle (120) eine weiße Leuchtdiode oder eine Glühlampe vorgesehen ist.

17. Laserbehandlungsgerät nach einem der vorhergehenden Ansprüche, **dadurch gekennzeichnet, daß** es ferner eine weitere Laserlichtquelle als Anregungslichtquelle für eine Fluoreszenz des zu behandelnden Materials umfaßt.

## Claims

1. A laser treatment appliance having an illumination system, in particular for use in the field of medicine, which has a laser light source (110) for producing a treatment laser beam (112) which is guided essentially freely, as well as an illuminating light source (120) for producing a visible light (122) for illuminating an area (50) to be treated, and one or more first optical elements (142, 144, 146, 148, 150, 152, 154, 156, 158, 172) via which the treatment laser beam (112) is passed on, with at least one of the first optical elements (142, 144, 146, 148, 150, 152, 154, 156, 158, 172) being in the form of an optical multifunction element (152, 154, 156, 158, 172), and such that the visible light (122) for illumination is also directed at least over this at least one optical multifunction element (152, 154, 156, 158, 172)
**characterized in that**
the laser treatment appliance furthermore has an optical evaluation apparatus (134) for diagnosis or analysis of radiation (130) which is produced during the treatment process, and all the optical multifunction elements (152, 154, 156, 158, 172) are designed such that they influence the treatment laser beam (112) and the visible illuminating light (122) essentially in an equivalent reflecting and/or transmitting manner, and the laser treatment appliance furthermore has a beam splitter (144), which extends at least over the entire cross section of the treatment laser beam (112), for decoupling the radiation (130), and which is arranged between the laser light source and the first multifunction element (152; 158) in the incidence direction of the treatment laser beam (112) such that the visible light (122) from the illuminating light source (130) does not pass through it.

2. The laser treatment appliance as claimed in claim 1, **characterized in that** said appliance is designed such that the treatment laser beam (112) is guided essentially freely in it. -

3. The laser treatment appliance as claimed in claim 1 or 2, **characterized in that** said appliance has a first optical element which is essentially in the form of a light outlet window (172) which transmits the laser treatment light and basically does not influence the profile of the treatment laser beam (112).

4. The laser treatment appliance as claimed in claim 3, **characterized in that** at least the light outlet window (172) is in the form of an optical multifunction element.

5. The laser treatment appliance as claimed in one of the preceding claims, **characterized in that** said appliance has at least one first optical element (152, 154, 156, 158), which influences the path of the treatment laser beam (112).

6. The laser treatment appliance as claimed in claim 5, **characterized in that** at least one of the first optical elements (152, 154, 156, 158) which influence the treatment laser beam is in the form of an optical multifunction element (152, 154, 156, 158).

7. The laser treatment appliance as claimed in claim 5 or 6, **characterized in that** at least one of the first optical elements which influence the treatment laser beam is a mirror element (154, 156, 158).

8. The laser treatment appliance as claimed in claim 5 to 7, **characterized in that** at least one of the first optical elements which influence the treatment laser beam is a focussing lens (152).

9. The laser treatment appliance as claimed in one of the preceding claims, **characterized in that** all the first optical elements are in the form of optical multifunction elements.

10. The laser treatment appliance as claimed in one of the preceding claims, **characterized in that** said appliance has a component (20) which has at least one optical element (152) for injecting the illuminating light (122) into the component (20).

11. The laser treatment appliance as claimed in claim 10, **characterized in that** the optical element (152) is designed for injecting the illuminating light (122) into the component such that the treatment laser beam (112) can also be injected via this element (152) into the component (20).

12. The laser treatment appliance as claimed in one of the preceding claims, **characterized in that** said appliance has a component which itself comprises the illuminating light source (120) and/or the laser light source.

13. The laser treatment appliance as claimed in one of claims 10 to 12, **characterized in that** the component is a handpiece (20) at the treatment end.

14. The laser treatment appliance as claimed in claim 13, **characterized in that** the handpiece (20) is designed such that the visible light (122) for illumination emerges from the handpiece (20) essentially in the same direction as the treatment laser beam (112).

15. The laser treatment appliance as claimed in one of the preceding claims, **characterized in that** said appliance is designed such that the beam axis of the treatment laser beam (112) on the one hand and the beam axis of the visible light (122) for illumination on the other hand are offset with respect to one another, at least in the first optical multifunction element (152; 158) in the emission direction.

16. The laser treatment appliance as claimed in one of the preceding claims, **characterized in that** a white light-emitting diode or an incandescent lamp is provided as the illuminating light source (120).

17. The laser treatment appliance as claimed in one of the preceding claims, **characterized in that** said appliance furthermore has a further laser light source as a stimulation light source for fluorescence of the material to be treated.

## Revendications

1. Appareil de traitement au laser, doté d'un système d'éclairage, en particulier pour l'utilisation dans le domaine médical, comprenant une source de lumière laser (110) pour produire un rayon laser d'usinage (112), guidé de façon sensiblement libre, ainsi qu'une source de lumière d'éclairage (120) pour produire une lumière visible (122) afin d'illuminer une zone (50) à usiner, et un ou plusieurs premiers éléments optiques (142, 144, 146, 148, 150, 152, 154, 156, 158, 172), à travers lesquels le rayon laser d'usinage (112) continue son chemin en étant guidé, au moins l'un des premiers éléments optiques (142, 144, 146, 148, 150, 152, 154, 156, 158, 172) étant formé en tant qu'élément optique multifonctionnel (152, 154, 156, 158, 172), et de manière que la lumière visible (122) servant à l'éclairage soit dirigée au moins également par l'intermédiaire de ce au moins un élément optique multifonctionnel (152, 154, 156, 158, 172), **caractérisé en ce que**
l'appareil de traitement au laser comprend en outre un dispositif d'évaluation optique (134) pour effectuer un diagnostic ou une analyse d'un rayonnement (130) produit lors d'un processus d'usinage, et tous les éléments optiques multifonctionnels (152, 154, 156, 158, 172) sont conçus de manière qu'ils influent sur le rayon laser d'usinage (112) et la lumière d'éclairage visible (122) sensiblement avec le même effet, par réflexion et/ou par transmission, et l'appareil de traitement au laser comprend en outre un diviseur de rayon (144) s'étendant au moins sur la totalité de la section transversale de l'appareil laser d'usinage (112) pour le désaccouplement du rayon (130), disposé entre la source de lumière laser et le premier élément multifonctionnel (152 ; 158) si l'on observe dans la direction d'incidence du rayon laser d'usinage (112), de manière ne pas être parcouru par la lumière visible (122) venant de la source de lumière d'éclairage (130).

2. Appareil de traitement au laser selon la revendication 1, **caractérisé en ce qu'**il est conçu de manière que le rayon de laser d'usinage (112) soit guidé de façon sensiblement librement lorsqu'il passe en lui.

3. Appareil de traitement au laser selon la revendication 1 ou 2, **caractérisé en ce qu'**il comprend un premier élément optique, réalisé essentiellement sous la forme de fenêtre de sortie de lumière (172), transmissive pour la lumière de traitement au laser et n'influant pratiquement pas sur l'allure du rayon laser d'usinage (112).

4. Appareil de traitement au laser selon la revendication 3, **caractérisé en ce qu'**au moins la fenêtre de sortie de lumière (172) est réalisée sous la forme d'élément multifonctionnel optique.

5. Appareil de traitement au laser selon l'une des revendications précédentes, **caractérisé en ce qu'**il comprend un premier élément optique (152, 154, 156, 158) influant sur l'allure du rayon laser d'usinage (112).

6. Appareil de traitement au laser selon la revendication 5, selon lequel au moins un premier élément optique (152, 154, 156, 158), influant sur le rayon laser d'usinage, est réalisé sous la forme d'élément multifonctionnel optique (152, 154, 156, 158).

7. Appareil de traitement au laser selon la revendication 5 ou 6, **caractérisé en ce qu'**au moins un premier élément optique, influant sur le rayon laser d'usinage, est un élément réfléchissant (154, 156, 158).

8. Appareil de traitement au laser selon les revendications 5 à 7, **caractérisé en ce qu'**au moins un premier élément optique, influant sur le rayon laser, d'usinage est une lentille de focalisation (152).

9. Appareil de traitement au laser selon l'une des revendications précédentes, **caractérisé en ce que** tous les premiers éléments optiques sont réalisés sous la forme d'éléments optiques multifonctionnels.

10. Appareil de traitement au laser selon l'une des revendications précédentes, **caractérisé en ce qu'**il présente un composant (20) comprenant au moins un élément optique (152) pour le couplage de la lumière d'éclairage (122) dans le composant (20).

11. Appareil de traitement au laser selon la revendication 10, **caractérisé en ce que** l'élément optique (152), servant au couplage de la lumière d'éclairage (122) dans le composant, est réalisé de manière à ce que, également, le rayon laser de traitement (112) peut être couplé au composant (20) par l'intermédiaire de cet élément (152).

12. Appareil de traitement au laser selon l'une des revendications précédentes, **caractérisé en ce qu'**il présente un composant, qui comprend lui-même la source de lumière d'éclairage (120) et/ou la source de lumière laser.

13. Appareil de traitement au laser selon l'une des revendications 10 à 12, **caractérisé en ce que** le composant est une pièce manuelle (20) située du côté de l'extrémité d'usinage.

14. Appareil de traitement au laser selon la revendication 13, **caractérisé en ce que** la pièce manuelle (20) est réalisée de manière que la lumière visible (122) servant à l'éclairage sorte sensiblement dans la même direction que le rayon laser d'usinage (112) sortant de la pièce manuelle (20).

15. Appareil de traitement au laser selon l'une des revendications précédentes, **caractérisé en ce qu'**il est réalisé de manière que l'axe de rayonnement du rayon laser d'usinage (112), d'une part, et l'axe de rayonnement de la lumière visible (122) pour l'éclairage, d'autre part, sont décalés l'un par rapport à l'autre, au moins dans le cas de l'élément multifonctionnel optique (152 ; 158) qui est le premier si l'on observe dans la direction d'émission du rayonnement.

16. Appareil de traitement au laser selon l'une des revendications précédentes, **caractérisé en ce qu'**est prévue comme source de lumière d'éclairage (122) une diode lumineuse blanche ou une lampe à incandescence.

17. Appareil de traitement au laser selon l'une des revendications précédentes, **caractérisé en ce qu'**il comprend en outre une autre source de lumière laser, en tant que source de lumière d'excitation pour une fluorescence du matériau à traiter.
